(19) Europäisches Patentamt
European Patent Office
Office européen des brevets

(11) **EP 2 854 626 B1**

(12) **EUROPÄISCHE PATENTSCHRIFT**

(45) Veröffentlichungstag und Bekanntmachung des Hinweises auf die Patenterteilung:
**03.06.2020 Patentblatt 2020/23**

(51) Int Cl.:
***A61B 5/0225*** *(2006.01)*

(21) Anmeldenummer: **13724786.2**

(22) Anmeldetag: **16.05.2013**

(86) Internationale Anmeldenummer:
**PCT/EP2013/060113**

(87) Internationale Veröffentlichungsnummer:
**WO 2013/178475 (05.12.2013 Gazette 2013/49)**

(54) **VERFAHREN UND VORRICHTUNG ZUR KONTINUIERLICHEN, NICHT-INVASIVEN BESTIMMUNG DES BLUTDRUCKES**

METHOD AND DEVICE FOR CONTINUOUS, NON-INVASIVE DETERMINATION OF BLOOD PRESSURE

PROCÉDÉ ET DISPOSITIF POUR DÉTERMINER LA PRESSION ARTÉRIELLE DE FAÇON CONTINUE ET NON INVASIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.05.2012 AT 502112012**

(43) Veröffentlichungstag der Anmeldung:
**08.04.2015 Patentblatt 2015/15**

(73) Patentinhaber: **CNSystems Medizintechnik AG**
**8020 Graz (AT)**

(72) Erfinder: **FORTIN, Jürgen**
**8043 Graz (AT)**

(74) Vertreter: **Babeluk, Michael**
**Patentanwalt**
**Florianigasse 26/3**
**1080 Wien (AT)**

(56) Entgegenhaltungen:
EP-A1- 2 319 408    US-A1- 2002 173 709
US-A1- 2005 148 885    US-A1- 2006 195 034
US-A1- 2007 032 729    US-A1- 2010 298 678

## Beschreibung

Die Erfindung betrifft ein Verfahren und eine Vorrichtung zur kontinuierlichen, nicht-invasiven Bestimmung des Blutdruckes mit Hilfe eines photoplethysmographischen Systems das zumindest eine Lichtquelle und zumindest einen Lichtdetektor aufweist, die an einem Körperteil angeordnet werden, der eine Arterie beinhaltet.

Die kontinuierliche nicht-invasive Messung des Blutdruckes stellt bis heute eine große Herausforderung an die Messtechnik dar. Am Markt beginnt sich die sog. "Vascular Unloading Technique" durchzusetzen, die auf eine Publikation von Penáz (Digest of the 10th International Conference on Medical and Biological Engineering 1973 Dresden) zurückgeht, bei der ein Finger durchleuchtet wird und durch eine Servoregelung der registrierte Fluss konstant gehalten wird.

Die photoplethysmographische Methode nach Penáz bzw. "Vascular Unloading Technique" oder in manchen Publikationen auch "Volume Clamp Methode" genannt, wurde weiter verbessert. Die EP 0 537 383 A1 (TNO) zeigt beispielsweise eine aufblasbare Fingermanschette für die nicht-invasive kontinuierliche Blutdrucküberprüfung. Der aufblasbare zylindrische Raum ist pneumatisch mit einer Fluidquelle verbunden. Eine Infrarotlichtquelle und ein Detektor sind beidseitig des Fingers innerhalb des festen Zylinders positioniert. Es ist ein Ventil zum Füllen des Zylinders mit Gas vorgesehen. Weiters sind elektrische Kabel für die Infrarotlichtquelle und des Detektors hindurchgeführt. Die US 4,510,940 A (Wesseling) und die US 4,539,997 A (Wesseling) zeigen Vorrichtungen zur kontinuierlichen nicht-invasiven Messung des Blutdruckes. Es sind eine fluidgefüllte Manschette, eine Lichtquelle, ein Lichtdetektor und ein Differenzdruckverstärker vorgesehen. Weiters zeigt die Veröffentlichung US 4,597,393 (Yamakoshi) eine Variante des Penáz-Prinzips.

In der WO 00/59369 A2 sind Verbesserungen der Ventilsteuerung bzw. des Druckerzeugungssystems sowie unterschiedliche Ausführungen der Druckmanschetten (z.B. Doppelmanschette) an verschiedenen Extremitäten dargestellt. In der WO 04/086963 A2 wird dargelegt, wie man die Doppelmanschette dahingehend nutzen kann, dass in einer Manschette der Blutdruck gemäß dem Penáz-Prinzip gemessen wird, wobei in der anderen Manschette eine optimierte Kontrolle des Arbeitspunktes (Setpoint SP) vorgenommen wird. Die WO 05/037097 A1 bzw. die US 2007/032729 A1 beschreibt ein verbessertes Regelungssystem für die Vascular Unloading Technique, wobei innen liegende Regelschleifen quasi optimierte Verhältnisse für die nächsten außen liegenden Regelschleifen darstellen. Die innere Regelschleife ermöglicht schnelle Druckänderungen, wobei für den Druckaufbau und den Druckabbau in einer Fingermanschette separate Einlass- und Auslassventile verwendet werden, um den Anpressdruck in Echtzeit dem arteriellen Blutdruck nachzuführen.

Die WO 2011/051822 A1 beschreibt wie man die Signalqualität der Vascular Unloading Technique verbessern kann, um dann in der weiteren Folge ein Verfahren der Pulskontur-Analyse für die Gewinnung weiterer Parameter anzuwenden. Die WO 2011/051819 A1 beschreibt ein verbessertes, ausschließlich digitales Verfahren und Gerät für die Vascular Unloading Technique.

Das Verfahren nach Penáz wurde in zahlreichen Patenten und Publikationen weiter entwickelt (siehe US 2006/0195034 A1 oder EP 2 319 408 A1), ein grundsätzlicher Nachteil des Verfahrens konnte jedoch nicht behoben werden: Für die Gewinnung des Blutdrucksignals muss ein Sensor vorzugsweise am Finger angebracht werden, dessen Anpressdruck in Echtzeit dem arteriellen Blutdruck im Finger nachgeführt werden muss. Diese rasche Drucknachführung kann nur mit dementsprechendem Aufwand realisiert werden. In allen bisherigen Veröffentlichungen wird dazu eine Manschette verwendet, die mit einer Pumpe sowie einem aufwendigen Ventil bzw. Ventilsystem verbunden ist. Der Innendruck der Manschette, der am Finger anliegt wird nun so geregelt, dass er dem arteriellen Blutdruck entspricht. Dies ist dann der Fall, wenn das gleichzeitig gemessene photoplethysmographische Signal konstant ist.

Der Manschettendruck muss idealer Weise so schnelle Veränderungen erlauben, wie sie im echten arteriellen Blutdruck auftreten können, d.h. er muss Änderungsfrequenzen bis etwa 20Hz zulassen. Dies stellt einen großen Aufwand für Ventil bzw. Ventilsystem, Pumpe und Manschette dar, die man sich gerne ersparen würde. Die vorliegende Erfindung soll diesen Aufwand erheblich reduzieren.

Aufgabe der Erfindung ist es ein Verfahren und eine Vorrichtung zur kontinuierlichen, nicht-invasiven Bestimmung des Blutdruckes bzw. des Blutdrucksignals $p_{BP}(t)$ [mmHg] vorzuschlagen das einfach realisierbar und unproblematisch anwendbar ist.

Wünschenswert wäre, wenn man nur ein photoplethysmographisches System ohne aufwendiges Drucksystem bräuchte. Ein photoplethysmographisches System besteht im Wesentlichen aus einer Lichtquelle (vorzugsweise LED) und einem Lichtdetektor (z.B. Fotodiode) und ist aus der Pulsoximetrie bestens bekannt. Das daraus gewonnene Signal $v(t)$ [dimensionslos, solange es nicht auf z.B. Liter kalibriert ist] ist ein Maß für das Volumen im Finger (Plethysmographie) - seine Pulsationen entsprechen dem arteriellen Blutvolumen. Der Gleichanteil des Signals wird durch die Dicke des Fingers und seine Gewebeanteile, durch das laminar strömende venöse Blut und andere Faktoren wie das Umgebungslicht bestimmt. Darüber hinaus enthält das photoplethysmographische Signal $v(t)$ aber auch veränderliche Anteile, die zumeist durch die Gefäßwand der Fingerarterie bestimmt werden. Fingerarterien gehören zu den Blutgefäßen, die durch vegetative Steuerung verengt (Vasokonstriktion) und erweitert (Vasodilatation) werden können. Diese vasomotorischen Veränderungen verändern das photoplethysmographische Signal derartig, dass es nicht direkt für die Blutdruckmessung verwendet werden kann.

Erfindungsgemäß wird diese Aufgabe dadurch gelöst,

- dass der Anpressdruck p(t) der Halterung des photoplethysmographischen Systems an den mittleren arteriellen Blutdruck MABP angepasst wird, indem

in einer Suchphase der initiale Anpressdruck $p_0$ der Halterung des photoplethysmographischen Systems bestimmt wird, bei dem das initiale photoplethysmographische Signal $V_0$ mit der größten Amplitude entsteht,

- dass der Anpressdruck p(t) der Halterung des photoplethysmographischen Systems ausgehend vom initialen Anpressdruck durch ein Regelsystem gesteuert wird,

- wobei die Änderungsgeschwindigkeit des Anpressdruckes der Halterung des photoplethysmographischen Systems kleiner ist als jene der pulsatilen Blutdruckänderungen in der Arterie, und

- wobei das Regelsystem ein gefiltertes photoplethysmographisches Signal vLF(t) erzeugt, welches mit dem initialen photoplethysmographischen Signal $V_0$ verglichen wird und der Anpressdruck p(t) so lange verändert wird, bis das gefilterte Signal vLF(t) wieder dem initialen Signal $V_0$ entspricht.

Eine erfindungsgemäße Vorrichtung - ausgehend von einem photoplethysmographischen System mit zumindest einer Lichtquelle und zumindest einem Lichtdetektor, die durch eine Halterung an einem eine Arterie enthaltenden Körperteil befestigt sind - zeichnet sich durch eine Einrichtung aus, die ein mechanisches, pneumatisches, elektromagnetisches oder elektromotorisches Stellwerk, wie z.B. einen Schrittmotor, einen Linearaktuator, etc. aufweist, mit der der Anpressdruck p(t) der Halterung an den Körperteil an den mittleren, arteriellen Blutdruck MABP anpassbar ist, indem in einer Suchphase der initiale Anpressdruck P der Halterung des photoplethysmographischen Systems bestimmt wird, bei dem das initiale photoplethysmographische Signal $V_0$ mit der größten Amplitude entsteht, wobei mit der Einrichtung eine Änderungsgeschwindigkeit des Anpressdruckes der Halterung des photoplethysmographischen Systems erzielbar ist, die kleiner ist als jene der pulsatilen Blutdruckänderungen in der Arterie des Körperteils, wobei die Vorrichtung ein Regelsystem aufweist, das geeignet ist ein gefiltertes photoplethysmographisches Signal $v_{LF}(t)$ zu erzeugen, welches mit einem initialen photoplethysmographischen Signal $V_0$ verglichen wird, wobei der Anpressdruck p(t) der Halterung so lange verändert wird, bis das gefilterte Signal $v_{FF}(t)$ wieder dem initialen Signal $V_0$ entspricht.

Das Besondere dabei ist, dass der Anpressdruck p(t) [mmHg] bzw. die Anpresskraft der Halterung und damit des plethysmographischen Systems dahingehend verändert wird, dass dieser bzw. diese auf den mittleren Blutdruck (Mean Arterial Blood Pressure MABP) abgestellt wird. Dieser mittlere Blutdruck MABP verändert sich im Verhältnis zum wahren pulsatilen intra-arteriellen Blutdruck $p_{BP}(t)$ relativ langsam - während man für Nachverfolgung des arteriellen Blutdruckes $p_{BP}(t)$ ein Druck- bzw. Manschettensystem braucht, das Drucksignale bis zu 20Hz verarbeiten muss, sind für die Nachführungen des mittleren Blutdruckes MABP Druckveränderungen weit unter den Frequenzen der Pulsationen ausreichend. Dies kann vorzugsweise mit einfachen mechanischen Systemen wie z.B. Schrittmotoren, Linearaktuatoren etc. erzielt werden, aber auch mit einfachen pneumatischen Systemen (Fingermanschetten) ohne aufwändige Ventilsysteme.

Für eine vorzugsweise adaptive Regelung ist es zunächst notwendig, den geeigneten Startpunkt zu finden. Dabei wird der Umstand ausgenützt, dass die Pulsationen des plethysmographischen Signals v(t) dann am größten sind, wenn der Anpressdruck p(t) dem mittleren Blutdruck MABP entspricht. Es wird daher zunächst der Anpressdruck p(t) solange verstellt, bis die Pulsationen am größten sind (Suchphase). Dann wird die adaptive Nachführung des Anpressdruckes p(t) aktiviert (Messphase). Das an dieser Stelle auftretende initiale plethysmographischen Signal $V_0$ wird für die nachfolgende Regelung gespeichert,
Die Regelung für die Drucknachführung basiert auf dem Umstand, dass zunächst das plethysmographische Signal v(t) so stark mit einem Tiefpass gefiltert wird, dass es für die Nachjustierung des Anpressdruckes verwendet werden kann. Diese gefilterte "Low Frequency LF" Signal $v_{LF}(t)$ wird mit dem initialen plethysmographischen Signal $V_0$ verglichen und der Anpressdruck p(t) so lange verändert, bis das gefilterte Signal $v_{LF}(t)$ wieder dem initialen Signal $V_0$ entspricht.

Für den Ausgleich der vasomotorischen Veränderungen wird der Umstand genutzt, dass beim mittleren Blutdruck die negative bzw. systolische Halbwelle von v(t) gleich groß ist wie die positive bzw. diastolische Halbwelle. Ist dies nicht der Fall, dann wird solange der Arbeitspunkt und dadurch der Druck nachgestellt, bis beide Halbwellen wieder gleich sind.

Das so gewonnene plethysmographische Signal v(t) entspricht noch nicht dem wirklichen Blutdrucksignal $p_{BP}(t)$, da die Absolutwerte des Blutdruckes so nicht bestimmt werden können. Deswegen wird der Blutdruck mit einem anderen intermittierenden Standardverfahren wie z.B. oszillometrisches Verfahren am Oberarm bestimmt und eine Transferfunktion für das photoplethysmographische Signal v(t) berechnet. Nach Anwendung dieser Transferfunktion auf das plethysmographische Signal v(t) liegt das kontinuierliche nicht-invasive Blutdrucksignal $p_{BP}(t)$ vor.

Die Erfindung wird im Folgenden anhand von schematischen Darstellungen und Diagrammen näher erläutert. Es zeigen:

Fig. 1 das Prinzip der Photoplethysmographie gemäß Stand der Technik,

Fig. 2 das Prinzip der "Vascular Unloading Technique" gemäß Stand der Technik,

Fig. 3 das erfindungsgemäße Messprinzip,

Fig. 4 ein Diagramm der zeitlichen Veränderung des photoplethysmographischen Signals v(t) bei Druckänderung p(t),

Fig. 5 ein Diagramm der S-förmigen Übertragungsfunktion zwischen Anpressdruck p und dem plethysmographischen Signal v(p),

Fig. 6 unterschiedliche plethysmographische Signale v(t) bei verschiedenen Anpressdrücken p anhand der S-förmigen Übertragungsfunktion,

Fig. 7 Veränderungen der S-förmigen Übertragungsfunktion und der plethysmographischen Signale bei Vasokonstriktion,

Fig. 8 eine Nachführung des Druckes auf der S-förmigen Übertragungsfunktion, sowie

Fig. 9 eine schematische Darstellung der erfindungsgemäßen Vorrichtung samt Regelung.

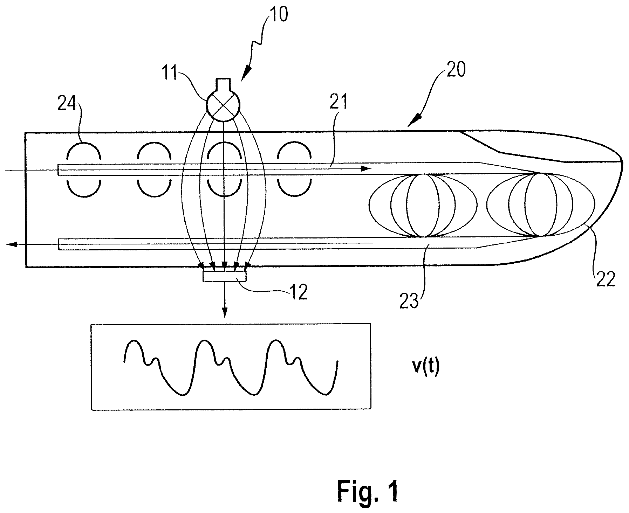

Fig. 1 zeigt das Prinzip der Photoplethysmographie. Die schematisch dargestellte Vorrichtung besteht im Wesentlichen aus einem photoplethysmographischen System 10 mit zumindest einer Lichtquelle 11 (z.B. eine LED) und zumindest einem Lichtdetektor 12, der ein photoplethysmographisches Signal v(t) erzeugt Durch den Körperteil, beispielsweise Finger 20, wird Licht gestrahlt, das primär durch arterielles Blut in der Arterie 21 absorbiert wird. Die Kapillargefäße sind mit 22, die Fingervene mit 23 bezeichnet. Weiters sind die pulsatilen Druckänderungen durch Ausbauchungen 24 der Arterie 21 angedeutet. Auf der anderen Seite des Fingers 20 wird das Restlicht vom Lichtdetektor 12 empfangen und in ein elektrisches Signal v(t) umgewandelt. Das Signal spiegelt so die arterielle Blutvolumenkurve bzw. den Durchmesser der Fingerarterie invertiert wider.

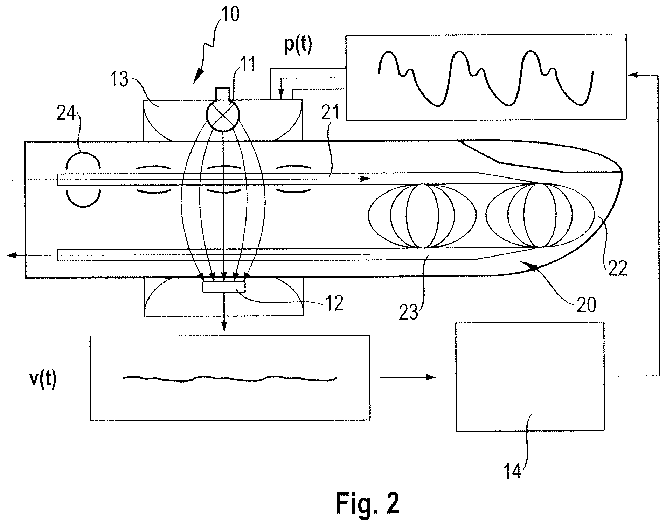

Fig. 2 beschreibt das Prinzip der "Vascular Unloading Technique". Um den Blutdruck nicht-invasiv und kontinuierlich messen zu können wurde die Vascular Unloading Technique entwickelt. Die Gefäßwand der Fingerarterie wird dadurch entspannt gehalten, indem ein Druck in einer anliegenden Halterung bzw. Manschette 13 so schnell geregelt wird, dass dieser Manschettendruck genau dem Druck in der Arterie 21 im Finger 20 entspricht. Das ist dann der Fall, wenn das dabei resultierende photoplethysmographische Signal v(t) konstant gehalten wird. Dieses Prinzip benötigt ein schnell reagierendes Druck- und Kontrollsystem 14, das vorzugsweise pneumatisch mit Pumpe, schnellen Ventil bzw. Ventilsystemen und der Fingermanschette 13 realisiert wird.

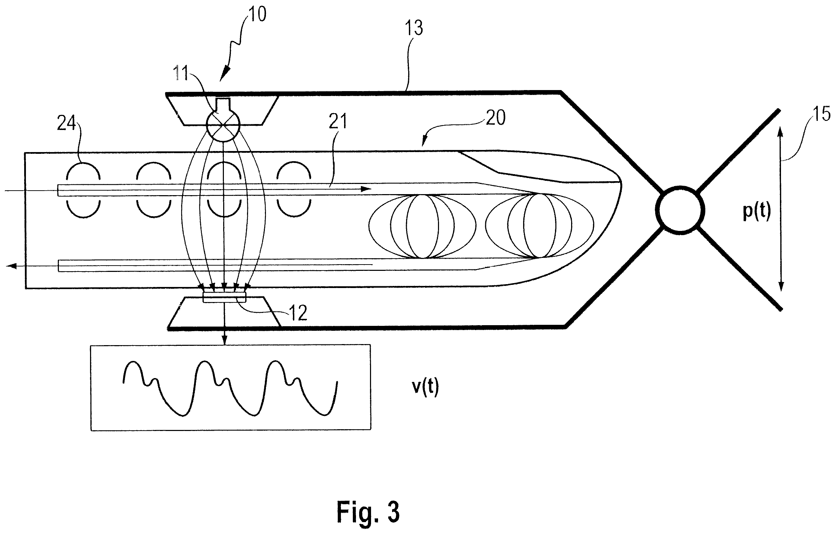

In Fig. 3 wird das erfindungsgemäße Messsystem dargestellt. Das schnell arbeitende Drucksystem der Vascular Unloading Technique ist sehr aufwendig und somit teuer. Die folgenden Überlegungen zeigen nun, dass es nicht notwendig ist, den Anpressdruck p(t) derart schnell nachzuführen, dass der wahre pulsatile, arterielle Blutdruck $p_{BP}(t)$ nachgebildet wird. Wichtig ist lediglich, den Anpressdruck p(t) dem mittleren Blutdruck MABP nachzuführen. Dies kann mit einer Drucknachführungsmethode geschehen, die viel langsamer bzw. träger ist, als die von der Vascular Unloading Technique bekannten Systeme. Fig. 3 zeigt eine Halterung 13 (z.B. Fingerklips) für die zumindest eine Lichtquelle 11 und den zumindest einen Lichtdetektor 12, um diese am Körperteil bzw. Finger 20 zu befestigen, der eine Arterie 21 aufweist. Erfindungsgemäß ist nun eine Einrichtung 15 vorgesehen, mit der der Anpressdruck der Halterung 13 an den Körperteil in Abhängigkeit vom mittleren Blutdruck veränderbar ist Die Nachführung kann so mit einem einfachen Schrittmotor oder Aktuator, aber auch Manschette mit einem langsamen Ventil bzw. Ventilsystem oder andere geeignete Vorrichtungen erfolgen.

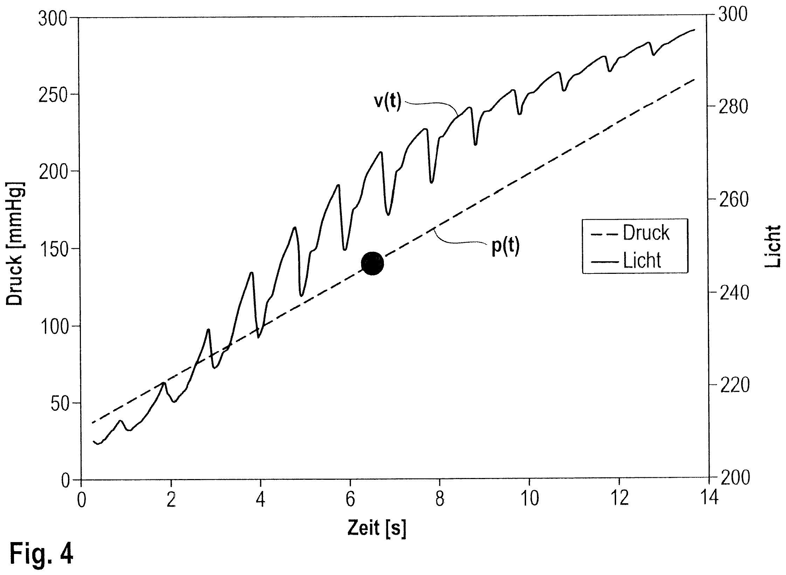

Bevor nun diese Nachführung beginnen kann, muss der initiale Anpressdruck $P_0$ der neuen Messvorrichtung auf den mittleren Blutdruck MABP gestellt werden. Es hat sich gezeigt, dass der Anpressdruck eines photoplethysmographischen Systems dann dem mittleren Blutdruckes MABP entspricht, wenn die Signalamplitude des photoplethysmographischen Signals v(t) am größten ist. In Fig. 4 wird gezeigt wie sich das photoplethysmographische Signal v(t) bei ansteigendem Druck p(t) ändert. Der schwarze Punkt markiert den Druck, bei dem die Amplitude von v(t) am größten ist - er entspricht dem aktuellen mittleren Blutdruck MABP. Dieser Arbeitspunkt - initialer Anpressdruck $P_0$ sowie initiales plethysmographisches Signal $V_0$ - wird weiters vom System in einem Speicher abgelegt. Man beachte dabei das invertierte Verhalten des Signals v(t), denn während der Systole befindet sich naturgemäß mehr Blut im Finger als bei der Diastole. Ein größeres Blutvolumen erhöht die Lichtabsorption und senkt somit das plethysmographische Signal während bei der Diastole das plethysmographische Signal wegen der geringeren Absorption steigt. Weiters wird durch den steigenden Druck immer mehr Blut aus dem Finger herausgepresst - auch deswegen steigt in Summe das plethysmographische Signal v(t) mit steigendem Druck p(t).

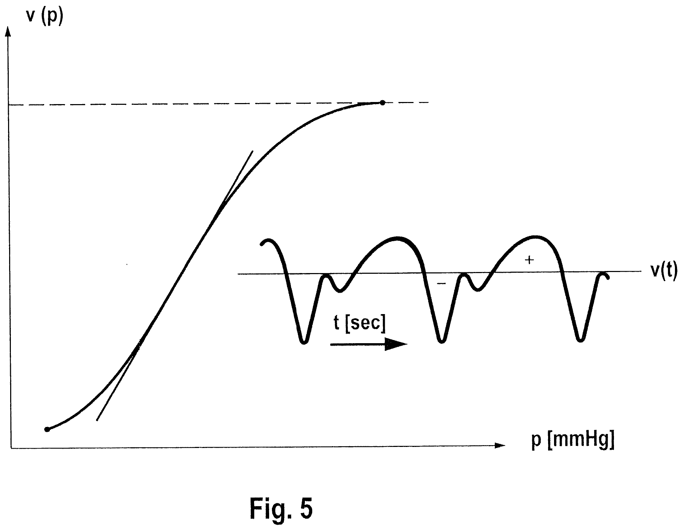

In Fig. 5 wird gezeigt, warum die Signalamplitude dann am größten ist, wenn der Anpressdruck genau dem mittleren Blutdruck MABP entspricht. Fig. 5 zeigt die S-förmige Übertragungsfunktion zwischen Anpressdruck p und dem plethysmographischen Signal v(p). Diese S-Kurve entsteht - in dem theoretischen Fall - wenn zunächst keine Pulsationen in der Arterie auftreten und das plethysmographische Signal v(p) versus dem Anpressdruck p aufgetragen wird. Durch die tatsächlichen arteriellen Pulsationen beginnt das plethysmographische Signal v(t) um den Arbeitspunkt zu schwingen, der durch den Anpressdruck eingestellt wird.

Die Amplitude des erzeugten plethysmographischen Signals v(t) wird durch die Steigung der S-Kurve bestimmt. In Fig. 5 entspricht der Arbeitspunkt dem Wendepunkt der S-Kurve, an dem die größte Steigung und somit die größte plethysmographische Amplitude feststellbar ist. Dieser Punkt entspricht dem mittleren Blutdruck MABP.

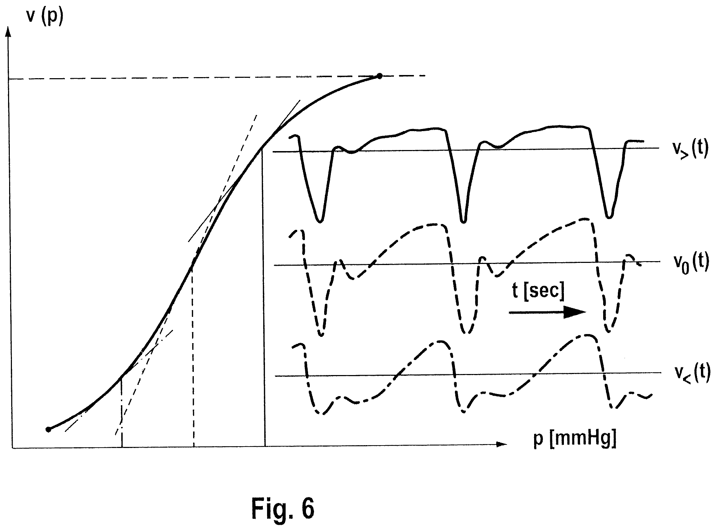

In Fig. 6 wird dargestellt, wie sich das plethysmographische Signal verändert wenn der Anpressdruck kleiner bzw. größer als der mittlere Blutdruck ist. Bei zu kleinem Anpressdruck entsteht das plethysmographische Signal $v_<(t)$. Im Unterschied zu $v_0(t)$, dem plethysmographischen Signal am Mitteldruck, ist die Amplitude kleiner, aber auch die Signalform ist verändert. Die Systole erscheint breiter, man kann die Signalform als "bauchiger" bezeichnen. Im Gegensatz dazu ist das plethysmographische Signal bei zu hohen Anpressdruck $v_>(t)$ "spitzer" als $v_0(t)$ sowie auch $v_<(t)$. Auch hier ist die Amplitude kleiner.

Nach der Suche der größten Amplitude, die bei dem Anpressdruck p(t) gefunden wird, der dem mittleren Blutdruck MABP bzw. der größten Steigung am Wendepunkt der S-Kurve entspricht steht das System am initialen Messpunkt. Dieser Arbeitspunkt soll nun in einer ersten Überlegung folgendermaßen beibehalten werden: Das initiale plethysmographische Signal $V_0$ als auch der initiale Anpressdruck $P_0$ werden vom System als der initiale Arbeitspunkt $V_0/P_0$ gespeichert.

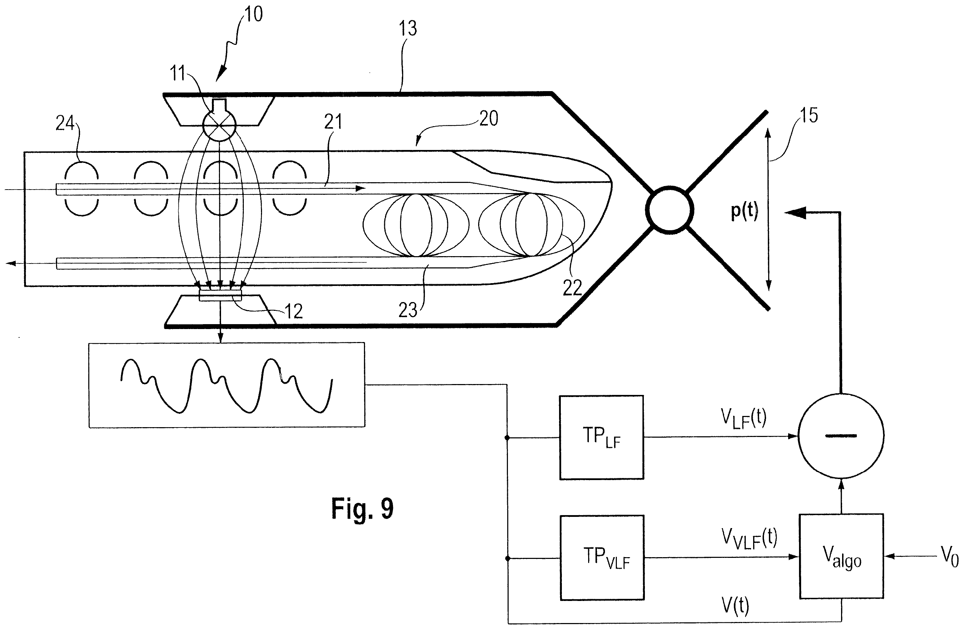

In weiterer Folge wird das plethysmographische Signal derart gefiltert, dass die pulsatilen Druckänderungen weitgehend verschwinden (siehe Filter TPLF in Fig. 9) und ein langsam veränderliches Signal $v_{LF}(t)$ entsteht, das von der erfindungsgemäßen Einrichtung 15 zur Druckänderung problemlos nachgestellt werden kann. In der Regel ist die Grenzfrequenz dieses Filters weit unter den Frequenzen der pulsatilen Druckänderungen. Dieses gefilterte Signal $v_{LF}(t)$ wird mit dem initialen Arbeitspunkt $V_0$ verglichen und bei einer Abweichung wird der Anpressdruck p(t) so lange nachgestellt, bis das Signal $V_0$ wieder erreicht wird.

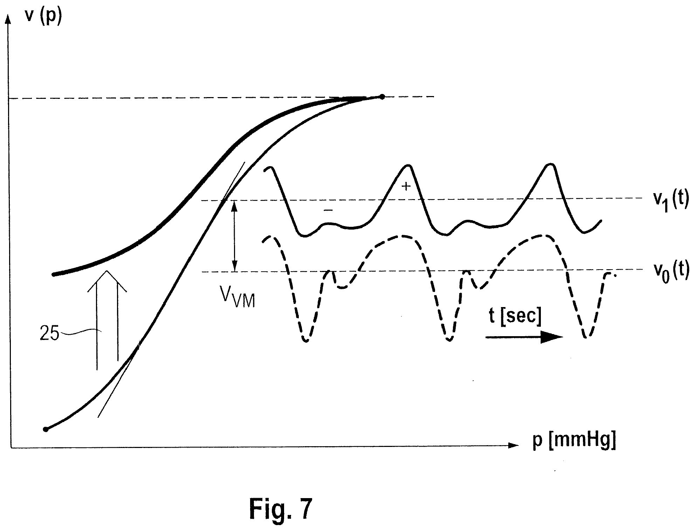

Eine solche Druckänderung entspricht im Diagramm der S-förmigen Übertragungsfunktion einer einfache Verschiebung der S-Kurve nach links bei höher werdendem Druck bzw. nach rechts bei niedriger werdendem Druck (nicht dargestellt). Leider kann es auch zu einer gleichzeitig auftretenden Verkleinerung der S-Kurve kommen, was einer Verengung der Arterie (sog. Vasokonstriktion gemäß Pfeil 25) entspricht (siehe Fig. 7). Bei einer Erweiterung der Arterie (sog. Vasodilatation) wird die S-Kurve analog zu Fig. 7 größer. Diese Veränderungen der S-förmigen Übertragungsfunktion durch vasomotorische Veränderungen (Vasokonstriktion bzw. Vasodilatation) können folgendermaßen berücksichtigt werden: Zunächst muss man beachten, dass diese Veränderungen nur sehr langsam auftreten und zwar im Minutenbereich. In diesem "Very Low Frequency VLF" Bereich kann nicht zwischen Blutdruckänderung oder Vasomotorik unterschieden werden und deswegen wird dieser Frequenzbereich durch Tiefpassfilterung (siehe Filter $TP_{VLF}$ in Fig. 9) mittels einem Signal $v_{VLF}(t)$ komplett entfernt. In der gegenständlichen Praxis bedeutet dies, dass der Vergleich mit dem initialen Arbeitspunkt $V_0$ nicht zulässig ist, weil sich dieser durch Vasokonstriktion nach oben bzw. durch Vasodilatation nach unter verschoben haben könnte.

Physiologisch passiert dabei folgendes: Durch Vasokonstriktion wird das Gefäß verengt und dadurch ist weniger Blut in der Arterie. Somit ist die Absorption geringer und das plethysmographische Signal wird größer, was einem Verschieben des Arbeitspunktes nach oben entspricht (siehe Fig. 7). Umgekehrt wird die Arterie bei Vasodilatation erweitert und mehr Blut befindet sich in der Arterie. Folglich nimmt die Absorption zu, das plethysmographische Signal sinkt und der Arbeitspunkt wandert nach unten.

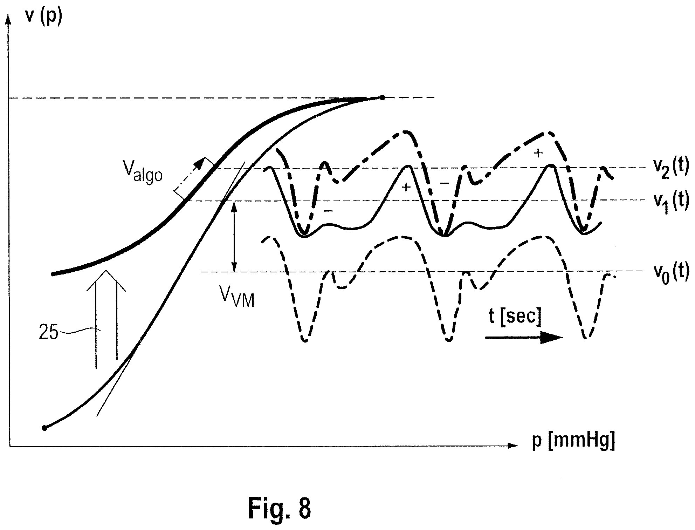

In Fig. 7 erkennt man, dass nicht nur der Arbeitspunkt wandert sondern auch die Signalform $v_1(t)$ verändert wird - sie wird "bauchiger" und entspricht dem Signal $v_<(t)$ bei zu geringen Anpressdruck. Weiters erkennt man das die negative Halbwelle (-) von $v_1(t)$ größer wird als die positive (+). Der Arbeitspunkt $V_0$ wird solange nachjustiert ($V_{algo}$), bis beide Halbwellen wieder gleich groß sind und $v_2(t)$ entsteht (Fig. 8). Die Veränderung von $V_0$ durch $V_{algo}$ ist nun der neue Arbeitspunkt für das gefilterte plethysmographische Signal. Der Anpressdruck p(t) wird so lange verändert bis $V_0$ plus $V_{algo}$ wieder gleich dem gefilterten plethysmographischen Signal $v_{LF}(t)$ ist.

Gemäß Fig. 9 weist die Regelung der erfindungsgemäßen Vorrichtung bevorzugt zwei Regelstrecken jeweils beginnend mit den Tiefpässen TPLF und TPVLF auf. Die eine verändert den Anpressdruck p(t) solange bis das gefilterte plethysmographische Signal $v_{LF}(t)$ einem Sollwert entspricht. Dieser Sollwert kommt aus der zweiten Regelstrecke und besteht einerseits aus dem initialen Arbeitspunkt $V_0$ plus einem adaptiven Wert $V_{algo}$. $V_{algo}$ wird erhöht, wenn die negative Halbwelle des ungefilterten plethysmographischen Signals $v(t) - v_{VLF}(t)$ größer als die positive Halbwelle ist. Sind beide Halbwellen gleich groß, bleibt $V_{algo}$ unverändert, ist die positive Halbwelle größer dann sinkt $V_{algo}$.

Damit aus diesen Signalen nun das kontinuierliche Blutdrucksignal berechnet werden kann muss eine Kalibrierung mit einem herkömmlichen Blutdruckmessgerät durchgeführt werden, das den systolischen sBP und diastolischen dBP Blutdruck ermittelt. In der Regel misst ein herkömmliches Blutdruckmessgerät den mittleren Blutdruck nicht, dieser kann jedoch gemäß der bekannten Formel

$$mBP = dBP + 0{,}33*(sBP{-}dBP)$$

ermittelt werden.

Weiters ist es von Vorteil, wenn die erfindungsgemäße Vorrichtung auch den wahren Anpressdruck $p(t)$ mittels einem Drucksensor misst, der ja dem mittleren Blutdruck entspricht. Das Blutdrucksignal $p_{BP}(t)$ kann dann folgendermaßen bestimmt werden:

$$p_{BP}(t) = mBP + p(t) - P_0 + (sBP{-}dBP)/(V_{0sys} - V_{0dia})*v(t)$$

wobei $V_{0sys}$ und $V_{0dia}$ dem systolischen bzw. diastolischen plethysmographischen Signal entspricht das während bzw. unmittelbar nach der Oberarmmessung aufgetreten ist.

Gemäß einer Ausführungsvariante kann eine direkte Messung des Anpressdruckes $p(t)$ mit einem Drucksensor unterbleiben, da man den Anpressdruck $p(t)$ auch aus der Stellung des Aktuators (beispielsweise aus der Stellung des Schrittmotors) aus der anfänglichen Suchphase ermitteln kann. Am Übergang von Suchphase zu Messphase steht ja bekanntlich der Anpressdruck $p(t)$ am mittleren Blutdruck MABP. Wird dann sofort der Oberarmblutdruck ermittelt, dann gilt ja:

$$p(t) = MABP = mPB = P_0.$$

Für den Verlauf der Messung und für den Verlauf des Anpressdrucks $p(t)$ gibt die Stellung des Schrittmotors - zumindest relativ - Auskunft.

Die Vorteile des neuen Messverfahrens bzw. der neuen Messvorrichtung bestehen zusammenfassend vor allem darin, dass der Anpressdruck $p(t)$ bzw. die Anpresskraft des photoplethysmographischen Systems auf den mittleren Blutdruck MABP geregelt wird. Dieser Druck verändert sich im Verhältnis zum wahren pulsatilen Blutdruck $p_{BP}(t)$ relativ langsam und kann vorzugsweise mit einfachen mechanischen Systemen wie z.B. Schrittmotoren, Linearaktuatoren aber auch einfache Fingermanschetten, ohne aufwändige Ventilsysteme erzielt werden. Die Regelung für die Drucknachführung basiert auf dem Umstand, dass zunächst das plethysmographische Signal so stark gefiltert wird, dass es für die Nachjustierung des Anpressdruckes verwendet werden kann, d.h. langsam genug für das träge mechanische Anpressdrucksystem ist. Dieses gefilterte Signal $v_{LF}(t)$ wird mit dem initialen plethysmographischen Signal $V_0$ verglichen und der Anpressdruck $p(t)$ so lange verändert, bis das gefilterte Signal $v_{LF}(t)$ wieder dem initialen Signal $V_0$ entspricht. Weiters ist ein Ausgleich für vasomotorische Veränderungen möglich. Das dabei entstehende plethysmographische Signal wird mit einem bekannten, intermittierenden Standardverfahren kalibriert.

**Patentansprüche**

1. Verfahren zur kontinuierlichen, nicht-invasiven Bestimmung des Blutdruckes mit Hilfe eines photoplethysmographischen Systems (10), wobei eine Halterung (13) mit zumindest einer Lichtquelle (11) und zumindest einem Lichtdetektor (12) an einem Körperteil (20) angeordnet wird, der eine Arterie (21) beinhaltet, **dadurch gekennzeichnet,**

   ▪ dass der Anpressdruck $p(t)$ der Halterung (13) des photoplethysmographischen Systems (10) an den mittleren arteriellen Blutdruck MABP angepasst wird, indem
   in einer Suchphase der initiale Anpressdruck $P_0$ der Halterung (13) des photoplethysmographischen Systems (10) bestimmt wird, bei dem das initiale photoplethysmographische Signal $V_0$ mit der größten Amplitude entsteht,
   ▪ dass der Anpressdruck $p(t)$ der Halterung (13) des photoplethysmographischen Systems (10) ausgehend vom initialen Anpressdruck durch ein Regelsystem gesteuert wird,
   ▪ wobei die Änderungsgeschwindigkeit des Anpressdruckes der Halterung (13) des photoplethysmographischen Systems (10) kleiner ist als jene der pulsatilen Blutdruckänderungen in der Arterie, und

- wobei das Regelsystem ein gefiltertes photoplethysmographisches Signal $v_{LF}(t)$ erzeugt, welches mit dem initialen photoplethysmographischen Signal $V_0$ verglichen wird und der Anpressdruck $p(t)$ so lange verändert wird, bis das gefilterte Signal $v_{LF}(t)$ wieder dem initialen Signal $V_0$ entspricht.

2. Verfahren nach Anspruch 1, **dadurch gekennzeichnet, dass** aus dem initialen Anpressdruck $P_0$ und dem initialen photoplethysmographischen Signal $V_0$ ein initialer Arbeitspunkt $(V_0/P_0)$ ermittelt und gespeichert wird.

3. Verfahren nach Anspruch 1 oder 2, **dadurch gekennzeichnet, dass** das Regelsystem physiologische Veränderungen der arteriellen Gefäßwand, beispielsweise vasomotorischen Veränderungen, durch eine Tiefpassfilterung des photoplethysmographische Signals berücksichtigt.

4. Verfahren nach Anspruch 3, **dadurch gekennzeichnet, dass** das Regelsystem physiologische Veränderungen der arteriellen Gefäßwand durch Vergleich der pulsatilen photoplethysmographischen Signalanteile erkennt.

5. Verfahren nach einem der Ansprüche 1 bis 4, **dadurch gekennzeichnet, dass** das aus der photoplethysmographischen Messung abgeleitete Blutdrucksignal mittels einer intermittierende Blutdruckmessung, z.B. einer Oberarmmessung, kalibriert wird.

6. Verfahren nach Anspruch 5, **dadurch gekennzeichnet, dass** für die Kalibration des Blutdrucksignals der mittlere Blutdruck der intermittierenden Oberarmmessung, der Anpressdruck der Halterung (13) des photoplethysmographischen Systems (10) sowie ggf. die pulsatilen photoplethysmographischen Signalanteile verwendet werden.

7. Verfahren nach einem der Ansprüche 1 bis 6, **dadurch gekennzeichnet, dass** der Anpressdruck der Halterung (13) aus der für die Nachführung notwendigen Stellgröße ermittelt wird.

8. Vorrichtung für die kontinuierliche, nicht-invasive Bestimmung des Blutdruckes mit einem photoplethysmographischen System (10), enthaltend

- zumindest eine Lichtquelle (11), vorzugsweise eine LED,
- zumindest einen Lichtdetektor (12), vorzugsweise eine Fotodiode, der bzw. die ein photoplethysmographisches Signal erzeugt, sowie
- eine Halterung (13), um die zumindest eine Lichtquelle (11) und den zumindest einen Lichtdetektor (12) an einem Körperteil (20) zu befestigen, der eine Arterie (21) enthält,

**dadurch gekennzeichnet, dass** eine Einrichtung (15) vorhanden ist, die ein mechanisches, pneumatisches, elektromagnetisches oder elektromotorisches Stellwerk, wie z.B. einen Schrittmotor, einen Linearaktuator, etc. aufweist, mit der der Anpressdruck $p(t)$ der Halterung (13) an den Körperteil (20) an den mittleren, arteriellen Blutdruck MABP anpassbar ist, indem in einer Suchphase der initiale Anpressdruck $P_0$ der Halterung des photoplethysmographischen Systems bestimmt wird, bei dem das initiale photoplethysmographische Signal $V_0$ mit der größten Amplitude entsteht, wobei mit
der Einrichtung (15) eine Änderungsgeschwindigkeit des Anpressdruckes der Halterung (13) des photoplethysmographischen Systems (10) erzielbar ist, die kleiner ist als jene der pulsatilen Blutdruckänderungen in der Arterie (21) des Körperteils (20), sowie dass die Vorrichtung ein Regelsystem aufweist, das geeignet ist ein gefiltertes photoplethysmographisches Signal $v_{LF}(t)$ zu erzeugen, welches mit einem initialen photoplethysmographischen Signal $V_0$ verglichen wird, wobei der Anpressdruck $p(t)$ der Halterung (13) so lange verändert wird, bis das gefilterte Signal $v_{LF}(t)$ wieder dem initialen Signal $V_0$ entspricht.

9. Vorrichtung nach Anspruch 8, **dadurch gekennzeichnet, dass** der Einrichtung (15) für die Änderung des Anpressdrucks ein Regelsystem zugeordnet ist, welches aus dem photoplethysmographischen Signal $v(t)$ des photoplethysmographischen Systems (10) mit einem ersten Filter (TPLF) ein von pulsatilen Druckänderungen gereinigtes Signal $v_{LF}(t)$ und mit einem zweiten Filter (TPVLF) ein von vasomotorischen Änderungen gereinigtes Signal $v_{VLF}(t)$ ableitet.

**Claims**

1. Method for the continuous, non-invasive determination of blood pressure by means of a photoplethysmographic system (10), wherein a mount (13) having at least one light source (11) and at least one light detector (12) is arranged on a body part (20) which contains an artery (21), **characterised in that**

- the contact pressure p(t) of the mount (13) of the photoplethysmographic system (10) is adapted to the mean arterial blood pressure MABP by determining, in a search phase, the initial contact pressure $P_0$ of the mount (13) of the photoplethysmographic system (10), at which the initial photoplethysmographic signal V0 with the greatest amplitude is produced,
- the contact pressure p(t) of the mount (13) of the photoplethysmographic system (10) is controlled by a control system starting from the initial contact pressure,
- wherein the rate of change of the contact pressure of the mount (13) of the photoplethysmographic system (10) is smaller than that of the pulsatile blood pressure changes in the artery, and
- wherein the control system generates a filtered photoplethysmographic signal vLF(t) which is compared with the initial photoplethysmographic signal V0 and the contact pressure p(t) is varied until the filtered signal vLF(t) again corresponds to the initial signal V0.

2. Method according to claim 1, **characterised in that** from the initial contact pressure $P_0$ and the initial photoplethysmographic signal $V_0$ an initial operating point $(V_0/P_0)$ is determined and stored.

3. Method according to claim 1 or 2, **characterised in that** the control system takes into account physiological changes of the arterial vessel wall, for example vasomotor changes, by low-pass filtering of the photoplethysmographic signal.

4. Method according to claim 3, **characterised in that** the control system detects physiological changes of the arterial vessel wall by comparing the pulsatile photoplethysmographic signal components.

5. Method according to one of claims 1 to 4, **characterised in that** the blood pressure signal derived from the photoplethysmographic measurement is calibrated by means of an intermittent blood pressure measurement, e.g. an upper arm measurement.

6. Method according to claim 5, **characterised in that** the mean blood pressure of the intermittent upper arm measurement, the contact pressure of the mount (13) of the photoplethysmographic system (10) as well as optionally the pulsatile photoplethysmographic signal components are used for the calibration of the blood pressure signal.

7. Method according to one of claims 1 to 6, **characterised in that** the contact pressure of the mount (13) is determined from the manipulated variable necessary for tracking.

8. Device for the continuous, non-invasive determination of blood pressure with a photoplethysmographic system (10), containing

- at least one light source (11), preferably an LED,
- at least one light detector (12), preferably a photodiode, which generates a photoplethysmographic signal, and
- a mount (13) for attaching the at least one light source (11) and the at least one light detector (12) to a body part (20) containing an artery (21),

**characterised in that** a device (15) is present which comprises a mechanical, pneumatic, electromagnetic or electromotive adjusting mechanism, such as a stepping motor, a linear actuator, etc. with which the contact pressure p(t) of the mount (13) on the body part (20) can be adapted to the mean arterial blood pressure MABP by determining in a search phase the initial contact pressure $P_0$ of the mount of the photoplethysmographic system, at which the initial photoplethysmographic signal $V_0$ with the greatest amplitude is produced, wherein a rate of change of the contact pressure of the mount (13) of the photoplethysmographic system (10) can be achieved with the device (15), which is smaller than that of the pulsatile blood pressure changes in the artery (21) of the body part (20), and **in that** the device has a control system which is suitable for generating a filtered photoplethysmographic signal $v_{LF}(t)$ which is compared with an initial photoplethysmographic signal $V_0$, wherein the contact pressure p(t) of the mount (13) is varied until the filtered signal $v_{LF}(t)$ again corresponds to the initial signal $V_0$.

9. Device according to claim 8, **characterised in that** the device (15) for changing the contact pressure is assigned a control system which derives from the photoplethysmographic signal v(t) of the photoplethysmographic system (10) with a first filter ($TP_{LF}$) a signal $v_{LF}(t)$ cleaned from pulsatile pressure changes and with a second filter ($TP_{VLF}$) a signal $v_{VLF}(t)$ cleaned from vasomotor changes.

**Revendications**

1. Procédé pour la détermination non invasive, en continu, de la pression sanguine à l'aide d'un système photopléthysmographique (10), avec un support (13) muni d'au moins une source lumineuse (11) et d'au moins un photodétecteur (12) appliqué sur une partie du corps (20) ayant une artère (21),
procédé **caractérisé en ce que**

- on adapte la pression d'application p(t) du support (13) du système photopléthysmographique (10) à la pression artérielle moyenne MABP **en ce que**
- dans une phase de recherche, on détermine la pression d'application initiale (Po) du support (13) du système photopléthysmographique (10), donnant le signal photopléthysmographique initial (Vo) ayant la plus grande amplitude
- on commande la pression d'application p(t) du support (13) du système photopléthysmographique (10) en partant de la pression d'application initiale à l'aide d'un système de régulation
- la vitesse de variation de la pression d'application du support (13) du système photopléthysmographique (10) étant inférieure à celle de la variation pulsée de la pression sanguine dans l'artère.
- le système de régulation générant un signal photopléthysmographique filtré $V_{LF}$ (t) qui est comparé au signal photopléthysmographique initial (Vo) et on modifie la pression d'application p(t) jusqu'à que le signal filtré $V_{LF}$ (t) corresponde de nouveau au signal initial (Vo).

2. Procédé selon la revendication 1,
**caractérisé en ce que**
à partir de la pression d'application initiale (Po) du support (13) et du signal photopléthysmographique initial (Vo), on déduit un point de fonctionnement initial (Vo/Po) et on l'enregistre en mémoire.

3. Procédé selon la revendication 1 ou 2,
**caractérisé en ce que**
le système de régulation tient compte des variations physiologiques de la paroi artérielle, par exemple des variations vasomotrices, par un filtrage-passe-bas du signal photopléthysmographique.

4. Procédé selon la revendication 3,
**caractérisé en ce que**
le système de régulation reconnaît les variations physiologiques de la paroi artérielle par la comparaison des composantes de signal photopléthysmographique pulsé.

5. Procédé selon l'une des revendications 1 à 4,
**caractérisé en ce que**
le signal de pression sanguine déduit de la mesure photopléthysmographique est calibré à l'aide d'une mesure intermittente de pression sanguine, par exemple d'une mesure à l'arrière-bras.

6. Procédé selon la revendication 5,
**caractérisé en ce que**
pour le calibrage du signal de pression sanguine, on utilise la pression sanguine moyenne de la mesure intermittente sur l'arrière-bras, la pression d'application du support (13) du système photopléthysmographique (10) ainsi que le cas échéant, des composantes de signal photopléthysmographique pulsé.

7. Procédé selon l'une des revendications 1 à 6,
**caractérisé en ce que**
on détermine la pression d'application du support (13) à partir de grandeurs de réglage nécessaires pour l'asservissement.

8. Dispositif pour la détermination non invasive, continue, de la pression sanguine à l'aide d'un système photopléthysmographique (10) comprenant

- au moins une source lumineuse (11) de préférence une LED,
- au moins un photodétecteur (12), de préférence une photodiode qui génère le ou les signaux photopléthysmographiques ainsi que
- un support (13) muni d'au moins une source lumineuse (11) et d'au moins un photodétecteur (12) fixé à une partie du corps (20) qui comporte une artère (21),

dispositif **caractérisé en ce qu'**il comporte

une installation (15) munie d'un dispositif d'actionnement mécanique, pneumatique, électromagnétique ou électromoteur, comme par exemple un moteur pas à pas, un actionneur linéaire etc., qui adapte la pression d'application du support (13) contre la partie du corps (20) à la pression sanguine artérielle moyenne MABP **en ce que** dans une phase de recherche on détermine la pression d'application initiale (Po) du support du système photopléthysmographique pour laquelle on a le signal photopléthysmographique initial (Vo) ayant la plus grande amplitude,
selon lequel

l'installation (15) fait varier la vitesse de la pression d'application du support (13) du système photopléthysmographique (10) qui est inférieure à celle des variations de pression sanguine pulsées dans l'artère (21) de la partie corps (20), et le dispositif comporte un système de régulation permettant de générer un signal photopléthysmographique filtré $V_{LF}$ (t) qui est comparé à un signal photopléthysmographique initial (Vo), la pression d'application p (t) du support (13) étant modifiée jusqu'à ce que le signal filtré $V_{LF}$ (t) corresponde de nouveau au signal (Vo).

9. Dispositif selon la revendication 8,
**caractérisé en ce que**
l'installation (15) comporte un système de régulation pour faire varier la pression d'application et à partir du signal photopléthysmographique (v(t)) du système photopléthysmographique (10), ce système déduit avec un premier filtre ($TP_{LF}$), un signal sans les variations pulsées de pression ($V_{LF}$ (t)), et avec un second filtre, un signal ($V_{VLF}$(t) sans les variations vasomotrices.

Fig. 1

Fig. 2

Fig. 3

EP 2 854 626 B1

Fig. 4

Fig. 5

EP 2 854 626 B1

Fig. 6

Fig. 7

Fig. 8

EP 2 854 626 B1

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0537383 A1
- US 4510940 A
- US 4539997 A
- US 4597393 A
- WO 0059369 A2
- WO 04086963 A2
- WO 05037097 A1
- US 2007032729 A1
- WO 2011051822 A1
- WO 2011051819 A1
- US 20060195034 A1
- EP 2319408 A1

**In der Beschreibung aufgeführte Nicht-Patentliteratur**

- **VON PEŇÁZ.** Vascular Unloading Technique. *Digest of the 10th International Conference on Medical and Biological Engineering,* 1973